# DEMANDE DE BREVET EUROPEEN

(11) **EP 1 525 860 A1**
(43) Date de publication de la demande: **27.04.2005**
(21) Numéro de dépôt: 03292664.4
(22) Date de dépôt: 24.10.2003
(51) Int. Cl.: A61F 2/14, A61F 9/007, A61F 9/00

(54) **Insert d'expansion sclerale**

(71) Demandeur: Gambini, Bernard, 15130 Giou de Mamou (FR)
(72) Inventeur: Gambini, Bernard, 15130 Giou de Mamou (FR)
(74) Mandataire: CAPRI

(57) **Abrégé**

Insert d'expansion sclérale comportant un pont épiscléral (7) en forme d'arche ayant deux pieds (72, 73) reliés par une entretoise (71), chaque pied ayant une surface d'extrémité adaptée à être placée en appui sur la sclère (1), l'insert étant disposé en totalité à l'extérieur de la sclère, l'entretoise (71) étant adaptée à recevoir au moins un pli d'une suture chirurgicale (8) plongeant dans la sclère.

## Description

La présente invention concerne un insert d'expansion sclérale pour l'ophtalmologie. Plus particulièrement, l'invention s'applique dans le traitement chirurgical de la presbytie. La presbytie est une affection qui touche le sujet autour de la quarantaine et qui se traduit par une difficulté, puis une impossibilité de la lecture de près. La présente invention a pour but de libérer la presbyte de la contrainte du port de lunettes ou de lentilles de contact.

Selon les nouvelles approches de la physio-pathogénie de la presbytie, cette dernière est due à un accroissement du diamètre du cristallin. Cette augmentation est due à l'âge. Le cristallin, enfermé dans un sac, est suspendu à la sclère, coque extérieure de l'oeil, le long d'un anneau de celle-ci, le muscle ou corps ciliaire, par un ligament appelé zonule. L'augmentation du diamètre du cristallin provoque une distension de la zonule. Les fibres zonulaires se détendent, rendant les contractions du muscle ciliaire moins efficaces pour tirer sur le cristallin.

On a déjà proposé de retendre les fibres de la zonule en augmentant le diamètre de la sclère situé à l'aplomb du corps ciliaire.

Le document FR-2 784 287 propose d'augmenter localement en plusieurs endroits le diamètre de l'anneau scléral au niveau du corps ciliaire de manière à retendre la zonule. Ceci est obtenu en disposant dans la sclère, des segments arqués que l'on met en place dans des passants incisés dans l'épaisseur de la sclère, chaque segment prenant appui sur la sclère par ses extrémités élargies tandis que la partie médiane du segment exerce sur le passant une force d'écartement vers l'extérieur. Pour faciliter la mise en place, chaque segment est en pratique formé de deux pièces, de façon à éviter d'avoir à passer une extrémité élargie du segment dans le passant incisé dans la sclère, et réduire la largeur de l'incision, et améliorer la stabilité du segment une fois mis en place. Cette mise en oeuvre présente plusieurs inconvénients. D'une part, elle oblige l'incision de la sclère, ce qui complique la mise en place de l'insert. De plus, la structure en deux parties complique aussi beaucoup cette mise en place, et présente des risques quant à la stabilité structurelle de l'insert. Les coûts de fabrication et de mise en place de l'insert sont donc également affectés de manière négative.

La présente invention a pour but de fournir un insert d'expansion sclérale qui ne reproduit pas les inconvénients susmentionnés.

Plus particulièrement, la présente invention a pour but de fournir un insert simple et peu coûteux à fabriquer et à mettre en place.

La présente invention a donc pour objet un insert d'expansion sclérale comportant un pont épiscléral en forme d'arche ayant deux pieds reliés par une entretoise, chaque pied ayant une surface d'extrémité adaptée à être placée en appui sur la sclère, l'insert étant disposé en totalité à l'extérieur de la sclère, l'entretoise étant adaptée à recevoir au moins un pli d'une suture chirurgicale plongeant dans la sclère.

Avantageusement, ledit insert est réalisé en une seule pièce monobloc.

Avantageusement, chaque pied comporte des extensions latérales s'étendant des deux côtés de ladite entretoise.

D'autres caractéristiques et avantages de l'invention apparaîtront plus clairement au cours de la description qui va suivre, donnée à titre d'exemple non limitatif en regard des dessins ci-joints, sur lesquels :
- la figure 1 est une vue en coupe de la partie avant supérieure d'un oeil, par un plan de profil passant par le centre de l'oeil ;
- la figure 2 est une vue en perspective d'un exemple d'insert conforme à l'invention,
- la figure 3 est une vue en coupe dans un plan passant sensiblement dans le plan de l'équateur du cristallin, un insert étant mis en place, et un fil de suture étant passé, mais non serré,
- la figure 4 est une vue analogue à la figure 3, après serrage du fil de suture,
- la figure 5 est une vue de face d'un oeil avec quatre inserts selon l'invention,
- la figure 6 est une vue en coupe analogue à la figure 3, et
- la figure 7 est une vue de face d'une partie de l'oeil avec un pont d'extension sclérale mis en place.

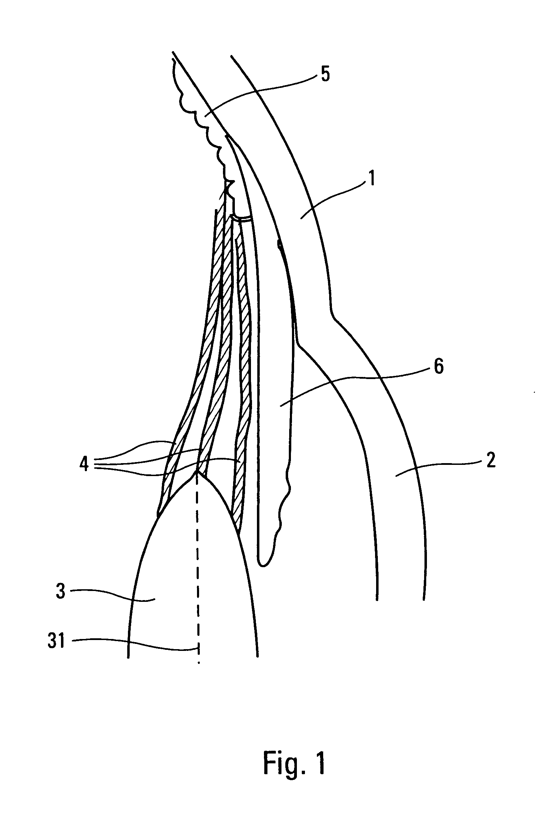

La figure 1 est une coupe de la partie avant supérieure d'un oeil par un plan de profil passant par le centre de l'oeil. On distingue la sclère 1 avec la cornée 2 transparente et une courbure plus accentuée. Le cristallin 3 est maintenu en place par la zonule 4 accrochée au cristallin au voisinage de son équateur 31 et au corps ou muscle ciliaire 5 qui s'étale le long de la sclère suivant une circonférence sensiblement concentrique au cristallin. On a également représenté l'iris 6.

Avec l'âge, les fibres zonulaires se détendent, rendant les contractions du muscle ciliaire moins efficaces pour tirer sur le cristallin. On a proposé de retendre les fibres de la zonule en augmentant le diamètre de la sclère situé à l'aplomb du corps ciliaire. Les moyens employés jusqu'à maintenant rendent nécessaire un découpage plus ou moins important de la sclère. Selon l'invention, on utilise un pont épiscléral 7, sans entamer la sclère autrement qu'en y passant des fils de suture chirurgicale.

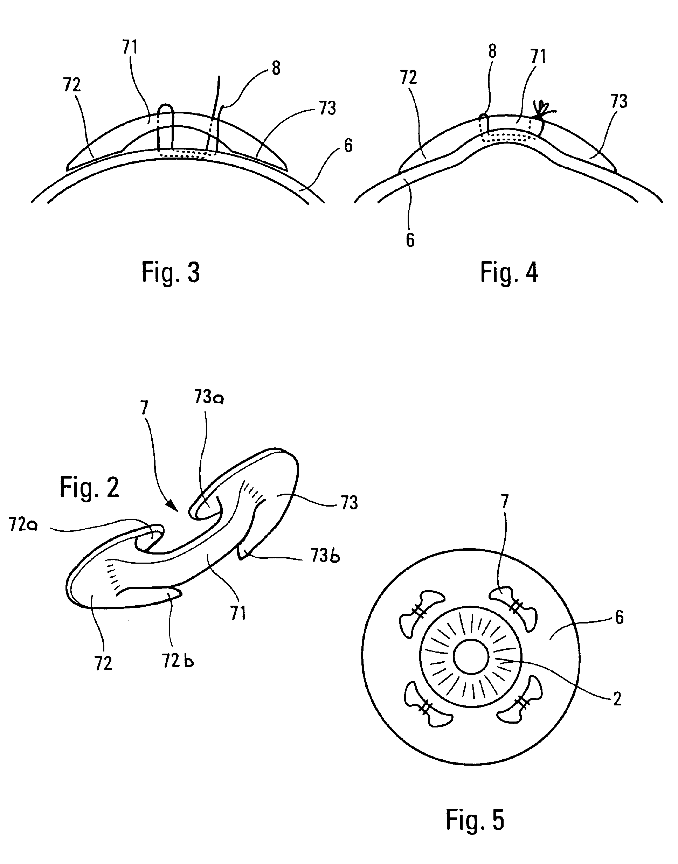

La figure 2 est une vue en perspective d'un exemple d'un insert selon l'invention. C'est un pont 7 formé d'une arche avec deux pieds 72 et 73 reliés par une entretoise 71.

Les figures 3 et 4 représentent schématiquement l'opération chirurgicale utilisant les moyens de l'invention. Ce sont des vues en coupe par un plan passant sensiblement par l'équateur du cristallin. On place sur la sclère 6, à l'aplomb du corps ciliaire le long de l'anneau situé dans le plan de l'équateur du cristallin un pont 7 tel que celui représenté sur la figure 2. Puis on passe une suture chirurgicale 8 (figure 3) dont le milieu est replié sur l'entretoise 71 du pont 7, les deux brins passent dans la sclère et ressortent à une petite distance, et sont levés de part et d'autre de l'entretoise 71. Comme visible sur les dessins, les sutures 8 ne transpercent avantageusement pas l'insert 7, mais entourent simplement l'entretoise 71 de celui-ci. Lors de la mise en tension du fil (figure 4) la suture fixe l'insert à l'oeil et provoque l'expansion de la sclère sous l'arche du pont. La forme et la surface supérieure de l'entretoise sont avantageusement réalisées de façon à bien recevoir et stabiliser la suture.

L'intérêt du dispositif de l'invention et de la méthode d'utilisation est son aspect peu invasif pour le patient et très rapide pour le chirurgien. L'insert peut être mis en place, et éventuellement retiré, très rapidement. Il n'y a pas de matériel placé dans l'épaisseur de la sclère (exceptée la suture), et pas de risque pour l'intégrité de l'oeil à long terme. On peut disposer un ou plusieurs inserts sur plusieurs espaces épiscléraux selon la l'importance de la presbytie.

La figure 5 représente, vue de face, la mise en place de quatre inserts 7 chez un presbyte de plus de 55 ans, sur la sclère 6, autour de la cornée 2.

L'insert 7 est de préférence monobloc. Ceci est quasi impossible avec l'insert du document FR-2 784 287, car sinon l'incision dans la sclère serait beaucoup trop importante pour laisser un pied dudit insert. L'insert 7 peut être réalisé avec de la résine transparente comme celle qui est utilisée par les ocularistes pour la fabrication des prothèses oculaires.

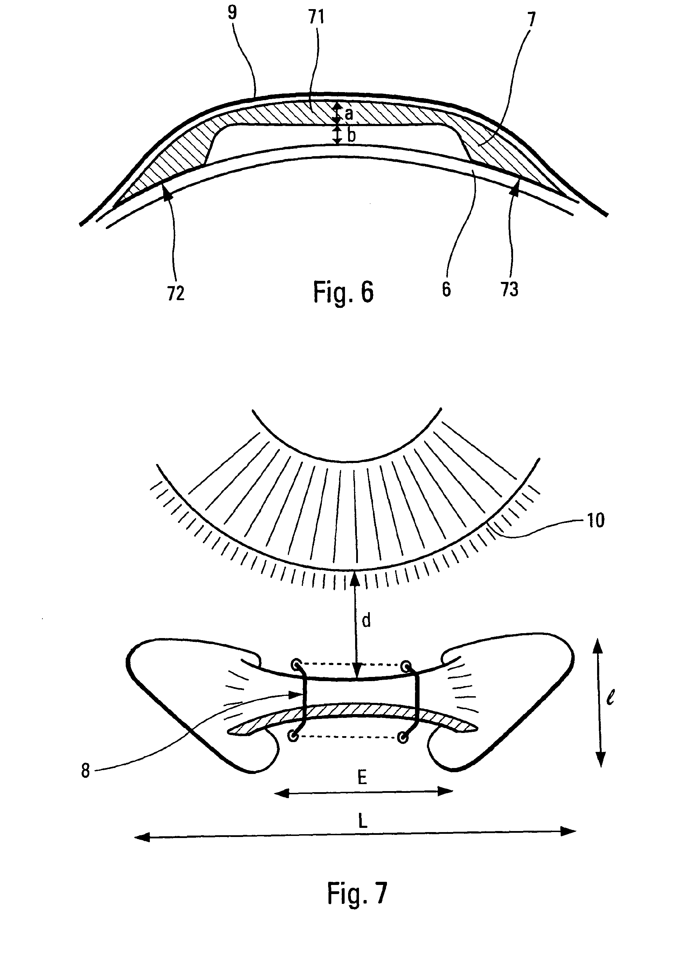

Les figures 6 et 7 représentent plus en détail un exemple de réalisation de l'invention. Le pont 7 en résine transparente est posé sur la sclère, et est placé sous la membrane conjonctive 9, qui couvre la sclère. La pose du pont est quasiment instantanée par une ouverture de la conjonctive. L'épaisseur « a » de l'entretoise 71 du pont 7 peut être de 1,5 mm, et l'écart « b » entre la sclère et l'entretoise, avant mise en place de la suture peut être de 1,5 mm. La sclère a en général une épaisseur voisine de 1 mm. Les pieds 72 et 73 ont une base concave, adaptée à la convexité de la sclère. Avantageusement, comme représenté schématiquement sur la figure 2, les pieds 72, 73 de l'insert 7 peuvent comporter des extensions latérales 72a, 72b, respectivement 73a, 73b, qui s'étendent des deux côtés de ladite entretoise 71, pour améliorer la stabilité de l'insert 7 et augmenter la surface d'appui de celui-ci sur la sclère 1. Ces extensions latérales pourraient même sensiblement se rejoindre, tout en laissant un espace pour la mise en place de la suture 8.

Le positionnement du pont est représenté plus en détail sur la vue de face de la figure 7. Le pont est placé à l'aplomb du corps ciliaire, à une distance « d » du limbe 10, frontière entre la cornée et la sclère qui peut être de 4 mm. La longueur L du pont peut être 11,5 mm et sa largeur 1 5 mm. La longueur E de l'entretoise peut être 4,5 mm.

Bien que la présente invention ait été décrite en référence à un mode de réalisation particulier de celle-ci, il est clair qu'elle n'est pas limitée par les exemples représentés. Au contraire, un homme du métier peut y apporter toutes modifications utiles sans sortir du cadre de la présente invention défini par les revendications annexées.

## Revendications

1. Insert d'expansion sclérale comportant un pont épiscléral (7) en forme d'arche ayant deux pieds (72, 73) reliés par une entretoise (71), chaque pied ayant une surface d'extrémité adaptée à être placée en appui sur la sclère (1), **caractérisé en ce que** l'insert est disposé en totalité à l'extérieur de la sclère, l'entretoise (71) étant adaptée à recevoir au moins un pli d'une suture chirurgicale (8) plongeant dans la sclère.

2. Insert selon la revendication 1, dans lequel ledit insert est réalisé en une seule pièce monobloc.

3. Insert selon la revendication 1 ou 2, dans lequel chaque pied (72, 73) comporte des extensions latérales (72a, 72b ; 73a, 73b) s'étendant des deux côtés de ladite entretoise (71).
